# EUROPEAN PATENT APPLICATION

(11) **EP 3 695 774 A1**
(43) Date of publication of application: **19.08.2020**
(21) Application number: 20155749.3
(22) Date of filing: 05.02.2020
(51) Int. Cl.: A61B 3/10, A61B 3/00

(54) **OPHTHALMIC DEVICE**

(30) Priority: 18.02.2019 JP 2019026715
(71) Applicant: Tomey Corporation, Nagoya-shi, Aichi 451-0051 (JP)
(72) Inventor: BIAN, Guangchun, Aichi-ken, 451-0051 (JP); TAKATA, Hideo, Aichi-ken, 451-0051 (JP); CHEN, Shaolang, Aichi-ken, 451-0051 (JP)
(74) Representative: BSB Patentanwälte Schütte & Engelen Partnerschaft mbB

(57) **Abstract**

Provided is a technique that makes it easy to specify the correspondence between an eye to be examined in an infrared image and an actual eye to be examined.

Configured is an ophthalmic device (1) including: an infrared photographing optical system that illuminates an eye to be examined with infrared rays to take an infrared image; a white light photographing optical system that illuminates the eye to be examined with white light to take a white light image; a photographing control unit (600a) that controls the infrared photographing optical system and the white light photographing optical system to take the infrared image and the white light image indicating the eye to be examined in the same state; and a display control unit (600b) that displays each of the infrared image and the white light image on a display unit (650).

## Description

The present invention relates to an ophthalmic device.

### Background of the Invention

Conventionally, an ophthalmic device that examines an eye to be examined based on an image taken by irradiating the eye to be examined with infrared rays is known. For example, Patent Literature 1 discloses a configuration in which light including infrared rays is emitted from a light source, and reflected light from a part to be observed is photographed with an infrared camera.

### Citation List

### Patent Literature

Patent Literature 1: JP 5281846 B

### Summary of the Invention

### Technical Problem

An examiner who operates the ophthalmic device usually observes the eye to be examined in a state where the eye to be examined is irradiated with visible light such as white light. Therefore, even if the examiner attempts to focus on a specific region such as a diseased region based on the infrared image taken in a state of being irradiated with infrared rays, it may be difficult to identify the specific region in the actual eye to be examined.

The present invention has been made in view of the above problems, and an object thereof is to make it easy to identify the correspondence between an eye to be examined in an infrared image and an actual eye to be examined. Solution to Problem

In order to achieve the above object, an ophthalmic device includes: an infrared photographing optical system that illuminates an eye to be examined with infrared rays to take an infrared image; a white light photographing optical system that illuminates the eye to be examined with white light to take a white light image; a photographing control unit that controls the infrared photographing optical system and the white light photographing optical system to take the infrared image and the white light image indicating the eye to be examined in the same state; and a display control unit that displays each of the infrared image and the white light image on a display unit.

Specifically, the ophthalmic device photographs the eye to be examined in the same state with infrared rays and white light, and displays each of the taken infrared image and white light image on the display unit. Therefore, in the ophthalmic device, the infrared image and the white light image are contrasted, thereby making it possible to easily associate the eyes to be examined in both the images with each other. Since the white light image is an image obtained by photographing the eye to be examined under white light, the color of the image of the eye to be examined within the white light image substantially matches the color of the eye to be examined visually recognized under white light. Therefore, if the eye to be examined in the infrared image and the eye to be examined in the white light image can be associated with each other, the correspondence between the eye to be examined in the infrared image and the actual eye to be examined can be easily identified.

### Brief Description of the Drawings

FIG. 1 is a diagram showing an optical system used at the time of measuring intraocular pressure.
FIG. 2 is a diagram showing an optical system used at the time of measuring eye refractive power, and an optical system used at the time of taking an infrared image and a white light image.
FIG. 3 is a block diagram illustrating the overall configuration of an ophthalmic device according to an Example of the present invention including control units.
FIG. 4A is a flowchart of photographing/display processing, and FIG. 4B is a flowchart of region-of-interest observation processing.
FIG. 5 is a diagram showing a display example of the infrared image and the white light image.
FIG. 6 is a diagram showing an example of a screen for performing image enhancement.

### Detailed Description of Preferred Embodiments

Embodiments of the present invention will be described in the following order.
(1) Configuration of ophthalmic device:
(2) Photographing/display processing:
(3) Region-of-interest observation processing:
(4) Other Embodiments:

### (1) Configuration of ophthalmic device:

An ophthalmic device 1 according to an embodiment of the present invention includes a casing, and optical systems and control units that are used for a plurality of types of observations and measurements are provided in the casing. In this embodiment, the ophthalmic device 1 has a function of displaying an image of an eye to be examined on a display unit to observe an abnormal region of a meibomian gland, a function of measuring eye refractive power, and a function of measuring intraocular pressure. In this embodiment, an optical system for observing an abnormal region of a meibomian gland, an optical system for measuring eye refractive power, and an optical system for measuring intraocular pressure share part of components.

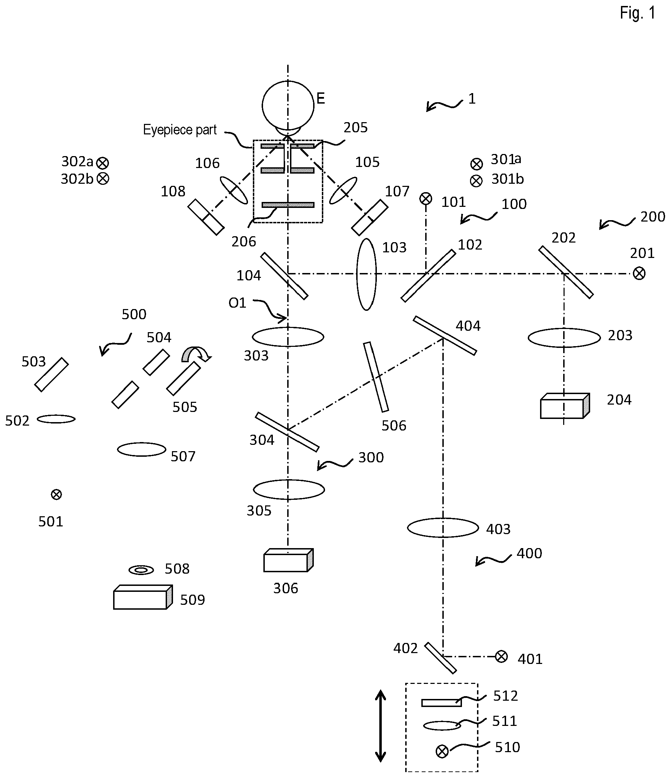

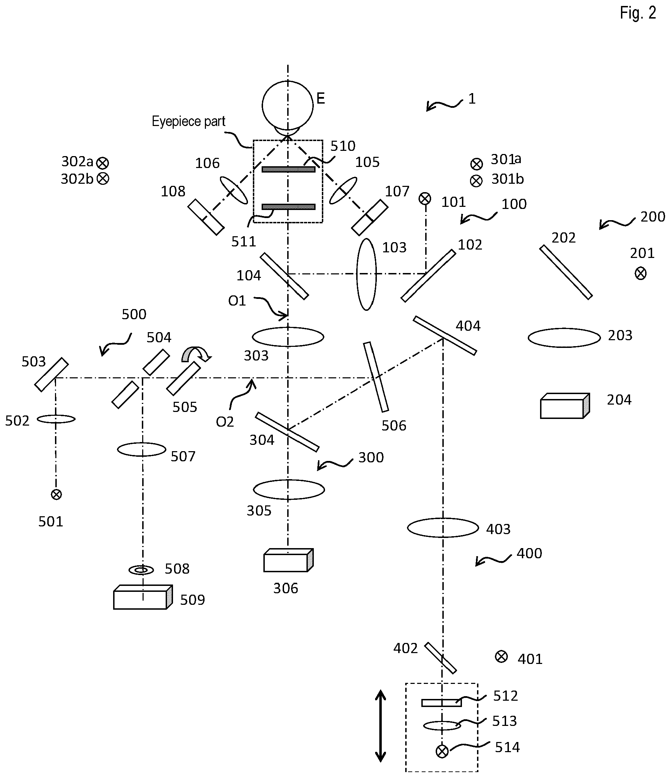

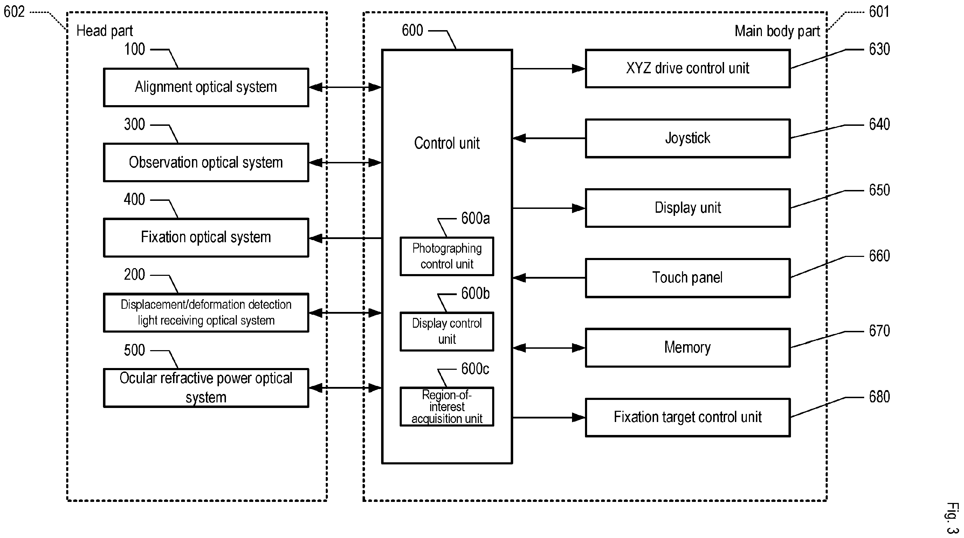

FIGS. 1 and 2 are diagrams showing these optical systems. FIG. 3 is a block diagram illustrating the overall configuration of the ophthalmic device 1 according to an Example of the present invention including control units. The ophthalmic device 1 according to an Example of the present invention will be described below with reference to FIGS. 1 to 3. As shown in FIG. 3, the ophthalmic device 1 is composed of: a head part 602 in which an optical system for measuring the eye to be examined is arranged; and a main body part 601 which includes a control unit 600 that controls, for example, the optical systems and the rotation motion of a switching unit in the head part 602.

Note that the main body part 601 is provided with: an XYZ drive control unit 630 that moves the head part 602 in XYZ (left and right, up and down, front and rear) directions relative to the main body part 601; a joystick 640 that instructs adjustment of the spatial position of the head part 602; a display unit 650 that displays an image of the photographed eye to be examined, and measurement results of the eye refractive power, the intraocular pressure and the like; a touch panel 660 that accepts instructions for measurement items and the like; a memory 670 that is used in the control processing of the control unit 600; and a fixation target control unit 680 that controls a fixation target unit (which will be described later).

### (Intraocular pressure measurement optical system)

FIG. 1 shows an overall optical system (intraocular pressure examination optical system) that measures the intraocular pressure of an eye to be examined. The intraocular pressure measurement optical system is provided with an alignment optical system 100 including optical elements or the like on optical paths from a light source 101 to profile sensors 107 and 108. The intraocular pressure measurement optical system is provided with an observation optical system 300 including optical elements or the like on optical paths from light sources 301a, 302a, 301b, and 302b to a two-dimensional image sensor (CCD) 306. Further, the intraocular pressure measurement optical system is provided with: a fixation optical system 400 including optical elements or the like on an optical path from a light source 401 through a reflection mirror 404 to an eye E to be examined; and a displacement/deformation detection light receiving optical system 200 that includes optical elements or the like on an optical path from a light source 201 to a nozzle 205 and detects the degree of deformation of the cornea of the eye to be examined. As shown in FIG. 1, a part of the respective optical systems constituting the intraocular pressure measurement optical system are configured to be shared. In this embodiment, it is possible to switch an eyepiece part arranged in front of the eye to be examined. When the intraocular pressure is measured, the switching unit is rotated to be positioned, and a nozzle 205 for measuring the intraocular pressure is arranged in front of the eye to be examined.

### (Alignment optical system 100)

In the alignment optical system 100, light from the light source 101 is reflected by a hot mirror 102, passes through an objective lens 103, is reflected by a hot mirror 104, then passes through a flat glass 206 and an opening of the nozzle 205, and applied to the cornea of the eye E to be examined. The light source 101 employed in this Example is an LED that outputs infrared light.

The light reflected by the cornea is received by a lens 105 and the profile sensor 107 as a first detection unit and a lens 106 and the profile sensor 108 as a second detection unit, which detection units are arranged symmetrically with respect to a main optical axis O1. In this embodiment, when the position of the eye to be examined in the three-dimensional direction is an appropriate position, the position where the light reflected by the cornea is detected by the profile sensor 107 and the profile sensor 108 is determined in advance. The control unit 600 of the main body part 601 instructs the XYZ drive control unit 630 to move the head unit 602 in the three-dimensional direction so that the light reflected by the cornea is detected by the profile sensor 107 and the profile sensor 108 and detected positions of the light the position determined in advance. As a result, the head part 602 and its internal optical system are aligned in the three-dimensional direction with respect to the eye to be examined.

Note that various techniques may be employed to perform alignment. For example, a configuration in which the control unit 600 performs auto-alignment (fine adjustment) after the examiner performs rough alignment can be employed. For the rough alignment, there can be employed, for example, a technique in which the examiner visually recognizes a bright spot due to the reflection from the cornea on the image of the eye to be examined displayed on the display unit 650, and further the joystick 640 is used to move the head part 602 so that the bright spot is positioned within a predetermined range. Of course, the alignment optical system is not limited to the examples as shown in FIGS. 1 and 2, and may be configured, for example, so that the optical system that performs alignment in the Z direction and the optical system that performs alignments in the XY directions are different optical systems which may partially overlap.

### (Observation optical system 300)

The observation optical system 300 is provided with light sources 301a, 302a, 301b, and 302b. In this embodiment, the light sources 301a and 302a are light sources that output infrared rays, and the light sources 301b and 302b are light sources that output white light. Note that the white light has only to have a spectral distribution such that the eye to be examined irradiated with the white light is visually recognized in full color, and the color temperature and the light intensity for each wavelength are not limited.

When the observation optical system 300 is used at the time of measuring the intraocular pressure, an anterior ocular region including the cornea part of the eye to be examined is irradiated by the light source 301a and the light source 302a arranged on the eye to be examined side of the head part 602. In this state, the anterior ocular image of the eye to be examined is acquired by an objective lens 303, an imaging lens 305, and the two-dimensional image sensor 306, and the acquired anterior ocular image of the eye to be examined is displayed on the display unit 650. LEDs that output infrared light are employed as the light sources 301a and 302a and the light source 101 for alignment. Light having a wavelength shorter than that of the light source 101 is employed as the light sources 301a and 302a. Therefore, the hot mirror 104 transmits light for observation (observation light) and reflects light for alignment (alignment light i.e. light from the light source 101). A dichroic mirror 304 has a reflection/transmission wavelength region set so that the observation light is transmitted. Thus, the alignment light and the observation light are appropriately divided to enable each measurement.

### (Fixation optical system 400)

When the fixation optical system 400 is used, light (fixation light) from the light source 401 is reflected by a hot mirror 402, passes through a relay lens 403, and is reflected by a reflection mirror 404. Thereafter, the light is transmitted through a hot mirror 506, is reflected by the dichroic mirror 304, passes through the main optical axis O1, passes through the objective lens 303 and the hot mirror 104, and forms an image on the retina of the eye to be examined. Therefore, it is desirable that the light source 401 and the position of the retina of the eye to be examined be substantially conjugate. The eye to be examined is fixated based on fixation light, and eye characteristics, such as intraocular pressure measurement, can be measured. As the light source 401, an LED that outputs visible light which can be visually recognized by a subject is employed.

### (Displacement/deformation detection light receiving optical system 200)

When the displacement/deformation detection light receiving optical system 200 is used, part of the light from the light source 201 (deformation detection light) is transmitted through a half mirror 202, then transmitted through the hot mirror 102 and the objective lens 103, reflected by the hot mirror 104, and passes through the main optical axis O1. Further, the light passes through the flat glass 206 and the opening of the nozzle 205 and is applied to the cornea of the eye to be examined. The light applied to the cornea is reflected by the cornea, and, in the reverse path, passes through the opening of the nozzle 205 and the flat glass 206, is reflected by the hot mirror 104, and passes through the objective lens 103 and the hot mirror 102, and part of the light is reflected by the half mirror 202. Thereafter, the light is received by a light receiving element 204 by a condenser lens 203. At the time of measuring the intraocular pressure, compressed air is blown toward the cornea of the eye to be examined from an air blow port provided at the tip of the nozzle 205. When air is blown, the cornea is displaced/deformed, so that the amount of light received by the light receiving element 204 changes. The intraocular pressure value of the eye to be examined is calculated from the degree of change in amount of light. An LED that outputs infrared light is also employed as the light source 201, but light having a wavelength longer than that of the observation light and a wavelength shorter than that of the alignment light is selected and employed. In this way, the wavelengths of the alignment light, the observation light, the fixation light, and the deformation detection light (light from the light source 201) are set, and the reflection/transmission characteristics of the hot mirrors 102, 104, 506, 402 and the dichroic mirror 304 are appropriately set, so that these four lights travel along appropriate optical paths.

### (Eye refractive power measurement optical system)

FIG. 2 shows an overall optical system (eye refractive power measurement optical system) that measures the eye refractive power of the eye to be examined. The eye refractive power measurement optical system is provided with the alignment optical system 100 including optical elements or the like on optical paths from the light source 101 to the profile sensors 107 and 108. The eye refractive power measurement optical system is provided with the observation optical system 300 including optical elements on optical paths or the like from the light sources 301a and 302a to the two-dimensional image sensor 306. Further, the eye refractive power measurement optical system is provided with the fixation optical system 400 including optical elements or the like on an optical path from a light source 514 through a fixation target 512 and the reflection mirror 404 to an eye E to be examined. Further, the eye refractive power measurement optical system is composed of an eye refractive power optical system 500 that includes optical elements or the like on an optical path from a light source 501 through a mirror 503 and a flat glass 511 to the eye E to be examined, and detects the eye refractive power of the eye to be examined. As shown in FIG. 2, a part of the respective optical systems constituting the eye refractive power measurement optical system are configured to be shared. When measuring the eye refractive power, a switching unit is rotated to be positioned, and flat glasses 510 and 511 for measuring the eye refractive power are arranged in front of the eye to be examined.

Since the alignment optical system 100 and the observation optical system 300 are the same as those at the time of the above-described intraocular pressure measurement, description thereof is omitted here. A fixation optical system 400 is partially different from that at the time of measuring the intraocular pressure, and will be described below.

### (Fixation optical system 400: eye refractive power measurement)

When measuring the eye refractive power, the light source 401 used at the time of measuring the intraocular pressure is turned off, and the light source 514, which is another light source, is turned on. The light from the light source 514 is collimated by a collimator lens 513 and applied to a fixation target 512. The light from the fixation target 512 is transmitted through the hot mirror 402 and the relay lens 403, then reflected by the reflection mirror 404, transmitted through the hot mirror 506, reflected by the dichroic mirror 304, and passes through the main optical axis O1. Thereafter, the light is transmitted through the objective lens 303, the hot mirror 104, and the flat glasses 511 and 510, and forms an image on the retina of the eye E to be examined. Therefore, it is desirable that the fixation target 512 and the position of the retina of the eye to be examined be substantially conjugate. The eye to be examined is fixated based on the fixation target 512. When measuring the eye refractive power, the control unit 600 outputs a control instruction to the fixation target control unit 680. As a result, the fixation target control unit 680 controls the movement of the fixation target unit (the fixation target 512, the collimator lens 513, and the light source 514) so that the fixation target and the position of the retina of the eye to be examined are substantially conjugate to fixate the eye to be examined. Thereafter, the control unit 600 outputs a control instruction to the fixation target control unit 680, and the fixation target control unit 680 moves the fixation target unit by a predetermined distance so as to establish a cloudy(foggy) state, and then measures the eye refractive power. Therefore, the fixation target unit can be moved frontward and rearward along the optical axis by a signal from the control unit 600. As the light source 514, an LED that outputs visible light which has a wavelength shorter than that of the light source 401 and can be visually recognized by the subject is employed.

### (Eye refractive power optical system 500)

When the eye refractive power optical system 500 is used, the light from the light source 501 (reflected light) is collected by a condenser lens 502, reflected by the mirror 503, and passes through a hole located at the center of a perforated mirror 504. Then, the light is transmitted through a parallel flat glass 505 that is arranged obliquely with respect to an optical axis O2 and rotates about the optical axis O2 by a driving unit (not shown), and then reflected by the hot mirror 506 and the dichroic mirror 304, and passes through the main optical axis O1. Then, the light is transmitted through the objective lens 303, the hot mirror 104, and the flat glasses 511 and 510 and is applied to the retina of the eye E to be examined. The reflected light from the retina of the eye E to be examined is transmitted through the flat glasses 510 and 511, the hot mirror 104, and the objective lens 303 through a path opposite to that during light application. Further, the light is reflected by the dichroic mirror 304 and the hot mirror 506, passes through the optical axis O2, is transmitted through the parallel flat glass 505, then reflected by the perforated mirror 504, and transmitted through a lens 507. Thereafter, the light forms an image in a ring shape (ring image) via a ring lens 508 by a two-dimensional image sensor (CCD) 509. The light source 501 employs infrared light having a wavelength longer than that of the alignment light (light source 101) and the observation light (light sources 301a and 302a). In this Example, an SLD (super luminescent diode) having a wavelength of 870 nm is employed. However, the light source is not limited to this, and an LED employed in the light source 101 or the like, or a laser diode (LD) may be employed.

Here, the parallel flat glass 505 is arranged at a position conjugate with the pupil of the eye E to be examined. When incident on the parallel flat glass 505 arranged obliquely with respect to the optical axis O2, the reflected light (light from the light source 501) is refracted and deviated by a predetermined distance (for example, ΔH) with respect to the optical axis O2. As described above, the parallel flat glass 505 rotates around the optical axis O2, and thus the reflected light transmitted through the parallel flat glass 505 rotates with a radius ΔH at a position of the parallel flat glass 505. Since the parallel flat glass 505 is arranged at a position conjugate with the position of the pupil of the eye E to be examined, the reflected light is applied to the retina of the eye to be examined while rotating with a predetermined (constant) radius (for example, Δh) at the position of the pupil of the eye E to be examined. Therefore, the reflected light forms, on the retina of the eye E to be examined, an image in a circular shape having a size and a shape corresponding to the eye refractive power of the eye E to be examined. Since the CCD 509 is arranged at a position conjugate with the retina of the eye E to be examined, the eye refractive power of the eye to be examined can be obtained by analyzing the ring image acquired by the CCD 509.

### (Infrared photographing optical system and white light photographing optical system)

In the ophthalmic device 1 according to this embodiment, in addition to the above measurements, it is possible to observe an abnormal region of a meibomian gland. An abnormal region of a meibomian gland is observed by photographing the eye to be examined using infrared rays and white light. In this embodiment, an abnormal region of a meibomian gland is observed with the eyepiece part being in the same state as in FIG. 2. That is, the eye to be examined is photographed with the back side of at least one of the lower eyelid and upper eyelid of the eye to be examined being exposed. In this embodiment, an infrared image is taken with the eye to be examined being illuminated with infrared rays, and, further, a white light image is taken with the eye to be examined being illuminated with white light.

When an infrared image is taken, the anterior ocular region including the cornea part of the eye to be examined is irradiated by the light source 301a and the light source 302a that output infrared rays. In this state, an infrared image of the eye to be examined is acquired by the objective lens 303, the imaging lens 305, and the two-dimensional image sensor 306. Therefore, in this embodiment, the infrared photographing optical system is provided with the light sources 301a and 302a, the objective lens 303, the imaging lens 305, and the two-dimensional image sensor 306. Note that the two-dimensional image sensor 306 is a general-purpose sensor that can detect light of each of red, green and blue colors, but can also detect the intensity of light within the infrared wavelength region by a specific color channel (for example, a channel for red) of the sensor.

When a white light image is taken, the anterior ocular region including the cornea part of the eye to be examined is irradiated by the light source 301b and the light source 302b that output white light. In this state, a white light image of the eye to be examined is acquired by the objective lens 303, the imaging lens 305, and the two-dimensional image sensor 306. Therefore, in this embodiment, the white light photographing optical system is provided with the light sources 301b and 302b, the objective lens 303, the imaging lens 305, and the two-dimensional image sensor 306. Thus, in this embodiment, the infrared photographing optical system and the white light photographing optical system share components other than the light sources. Therefore, the head part 602 can have a simple configuration, and images of the eye to be examined in the same state (which will be described later) can be easily taken.

The control unit 600 functions as a photographing control unit 600a and a display control unit 600b by executing control programs (not shown). The photographing control unit 600a causes the control unit 600 to execute a function of controlling the infrared photographing optical system and the white light photographing optical system included in the observation optical system 300, and to execute a function of taking an infrared image and a white light image showing the eye to be examined in the same state.

Specifically, in this embodiment, the observation optical system 300 is provided with a lens driving unit (not shown). The control unit 600 can control the driving unit by the function of the photographing control unit 600a to move at least one of the objective lens 303 and the imaging lens 305 in the optical axis direction. As a result, the control unit 600 can focus the eye to be examined on the two-dimensional image sensor 306.

Further, the control unit 600 can control the photographing timing of the two-dimensional image sensor 306 by the function of the photographing control unit 600a. The taken infrared image and white light image are acquired by the control unit 600 and recorded in the memory 670. Note that the objective lens 303 and the imaging lens 305 may be composed of various lenses, and the number of lenses is not limited.

The display control unit 600b causes the control unit 600 to execute a function of displaying each of the infrared image and the white light image on the display unit 650. That is, the control unit 600 acquires the infrared image and white light image recorded in the memory 670 and causes the display unit 650 to display them.

In this embodiment, the eye to be examined is photographed with the back side of at least one of the lower eyelid and upper eyelid of the eye to be examined being exposed, and the taken infrared image is displayed on the display unit 650, thereby enabling observation of the meibomian gland of a subject. That is, the meibomian gland, which is an organ secreting sebum, becomes clearer under infrared illumination than under white light illumination. Therefore, the infrared image displayed on the display unit 650 makes it easy to observe the meibomian gland and to observe an abnormality of the meibomian gland.

However, when the examiner attempts to identify and observe a region of interest in the infrared image under the white light in the actual eye to be examined, the region of interest may not be accurately identified. That is, since the image of the eye to be examined in the infrared image looks different from the eye to be examined under white light, it is generally difficult to identify the region of interest in the infrared image in the eye to be examined under white light. Therefore, this embodiment is configured so that the eye to be examined is photographed even under white light illumination, and that each of the infrared image and the white light image is displayed on the display unit.

Further, if the image of the eye to be examined in the infrared image is different from the image of the eye to be examined in the white light image, it is difficult to contrast the two images with each other to identify the region of interest in the infrared image using the white light image. Therefore, this embodiment is configured to take an infrared image and a white light image showing the eye to be examined in the same state.

### (2) Photographing/display processing:

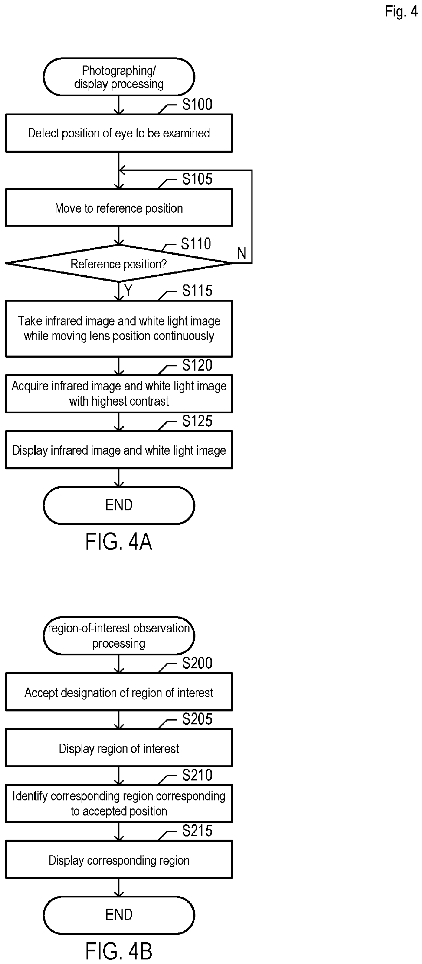

Hereinafter, photographing/display processing for photographing and displaying an infrared image and a white light image will be described. FIG. 4A is a flowchart showing photographing/display processing. When the subject sets his/her head at a predetermined position of the head part 602, the examiner exposes the back side of at least one of the lower eyelid and upper eyelid of the eye to be examined. In this state, when the examiner operates a touch panel 660 to instruct start of observation of a meibomian gland, the control unit 600 starts the photographing/display processing. When the photographing/display processing is started, the control unit 600 starts an alignment motion.

In the alignment motion, the control unit 600 first detects the position of the eye to be examined (step S100). Specifically, the control unit 600 turns on the light source 101 by the function of the photographing control unit 600a. As a result, the infrared light from the light source 101 is applied to the cornea of the eye E to be examined through the hot mirror 102, the objective lens 103, and the hot mirror 104. The infrared light is reflected by the cornea, and received by the profile sensor 107 via the lens 105 and received by the profile sensor 108 via the lens 106. The control unit 600 detects the position of the eye E to be examined based on the reflected light received by the profile sensors 107 and 108.

Next, the control unit 600 moves the head part 602 to a reference position (step S105). That is, the control unit 600 instructs the XYZ drive control unit 630 to move the head part 602. At this time, the control unit 600 instructs the XYZ drive control unit 630 so that the head part 602 moves by a predetermined amount toward the reference position determined in advance as the position where the eye E to be examined can be photographed. Specifically, when the head part 602 exists at the reference position, the positions where the reflected light from the cornea is received on the profile sensors 107 and 108 are determined in advance. Therefore, the control unit 600 moves the head part 602 by a predetermined amount so that the reflected light from the cornea moves toward the position determined in advance.

Next, the control unit 600 determines whether the head part 602 has reached the reference position (step S110). That is, the control unit 600 determines that the head part 602 has reached the reference position when the positions of the reflected light received by the profile sensors 107 and 108 are the positions determined in advance. When it is not determined in step S110 that the head part 602 has reached the reference position, the control unit 600 repeats the processing in step S105 and the subsequent steps.

On the other hand, when it is determined in step S110 that the head part 602 has reached the reference position, the control unit 600 takes an infrared image and a white light image while continuously moving the lens position by the function of the photographing control unit 600a (step S115). That is, the control unit 600 controls the lens driving unit in the observation optical system 300 by the function of the photographing control unit 600a, to move at least one of the objective lens 303 and the imaging lens 305 in the optical axis direction. In this embodiment, the movement of the lens is a scanning motion within a predetermined range, and the lens is moved so that the focusing position by the lens moves in one direction along the optical axis direction from an initial position to an end position. The lens is moved by a predetermined step each time an infrared image and a white light image are taken.

Therefore, first, the control unit 600 sets the lens at the initial position. Further, the control unit 600 controls the observation optical system 300 by the function of the photographing control unit 600a to turn on the light sources 301a and 302a that output infrared rays and to turn off the light sources 301b and 302b that output white light. Further, the control unit 600 controls the observation optical system 300 by using the function of the photographing control unit 600a to take an infrared image with the two-dimensional image sensor 306, and the infrared image is recorded in the memory 670. That is, the control unit 600 acquires the infrared intensity detected in each pixel based on the output of the two-dimensional image sensor 306, and the image data showing the gradation value indicating the intensity of infrared rays for each pixel is recorded as infrared image data in the memory 670.

Further, the control unit 600 controls the observation optical system 300 by the function of the photographing control unit 600a to turn off the light sources 301a and 302a that output infrared rays, and to turn on the light sources 301b and 302b that output white light. Further, the control unit 600 controls the observation optical system 300 by using the function of the photographing control unit 600a to take a white light image with the two-dimensional image sensor 306, and the white light image is recorded in the memory 670. That is, the control unit 600 acquires the light intensity of each color detected in each pixel based on the output for each color channel of the two-dimensional image sensor 306, and the image data showing the gradation value indicating the intensity of light of each color for each pixel is recorded as white light image data in the memory 670.

When the infrared image and the white light image are taken, the control unit 600 controls the observation optical system 300 by the function of the photographing control unit 600a to move at least one of the objective lens 303 and the imaging lens 305 by the predetermined step. Thereafter, the infrared image and the white light image are taken again, and, thereafter, the movement of the lens by the predetermined step and the taking of the infrared image and the white light image are repeated to the end position of the lens.

In the above photographing, the interval between the taking of the infrared image and the taking of the white light image, which are performed at the same lens position, is a period of one frame. In this embodiment, the two-dimensional image sensor 306 is an element capable of taking the images at 60 fps, and the period of one frame is 1/60 seconds. Therefore, these infrared image and white light image are taken almost simultaneously. For this reason, it can be said that the eye to be examined in the infrared image and the eye to be examined in the white light image, which are taken during the period when the lens position is identical, are in the same state.

In addition, the lens position is moved in a period of about one frame, and an image is taken immediately after the movement. Therefore, it can be said that the eye to be examined in the infrared image and the eye to be examined in the white light image, which are taken while the lens position is moved by several steps, are in the same state. In other words, when the subject's head is fixed onto the head part 602 and the examiner takes an image with the back of the subject's eyelid being exposed, for example, for about 0.5 seconds to 1 second, the eye to be examined hardly moves during this period. Therefore, it can be said that the infrared image and the white light image taken in such a period presents the photographing results of the eye to be examined in the same state.

Next, the control unit 600 acquires an infrared image and a white light image with the highest contrast by using the function of the photographing control unit 600a (step S120). In other words, in this embodiment, the images with the highest contrast are regarded as images taken in a focused state. Therefore, the control unit 600 specifies the contrast of each of the infrared images and the white light images recorded in the memory 670, and acquires one infrared image and one white light image having the highest contrast.

Needless to say, various known techniques may be employed to specify the contrast, and various other configurations may be employed as the configuration for acquiring the image in the focused state. For example, the control unit 600 may specify the position with the highest contrast in each of the infrared images and the white light images while moving the lens position, and acquire the infrared image and the white light image taken at the position.

When the infrared image and white light image with the highest contrast are acquired in the above way, each image is an image taken in the focused state, and thus the eye to be examined can be clearly recognized. The observation optical systems 300 for taking the infrared image and the white light image share optical systems other than the light sources, and the wavelengths of the infrared rays and the white light are close to each other. Therefore, the infrared image and the white light image with the highest contrast are images that are taken at the same or substantially the same lens position. Therefore, it can be said that the infrared image and the white light image acquired in step S120 present the photographing results of the eye to be examined in the same state.

Next, the control unit 600 displays the infrared image and the white light image by the function of the display control unit 600b (step S125). That is, the control unit 600 controls the display unit 650 to display the infrared image and white light image acquired in step S120. In this embodiment, the control unit 600 causes the display unit 650 to display the infrared image as a monochrome image and causes the display unit 650 to display the white light image as a color image.

That is, the image data of the infrared image acquired in step S120 is data showing the intensity of infrared rays for each pixel. Therefore, the control unit 600 generates a monochrome image on the assumption that the magnitude of the gradation value of each pixel represents luminance, and causes the display unit 650 to display the monochrome image. On the other hand, the image data of the white light image acquired in step S120 is data showing the intensity of light for each color channel for each pixel. Therefore, the control unit 600 generates a color image on the assumption that the magnitude of the gradation value for each color of each pixel represents the intensity of light of each color, and causes the display unit 650 to display the color image.

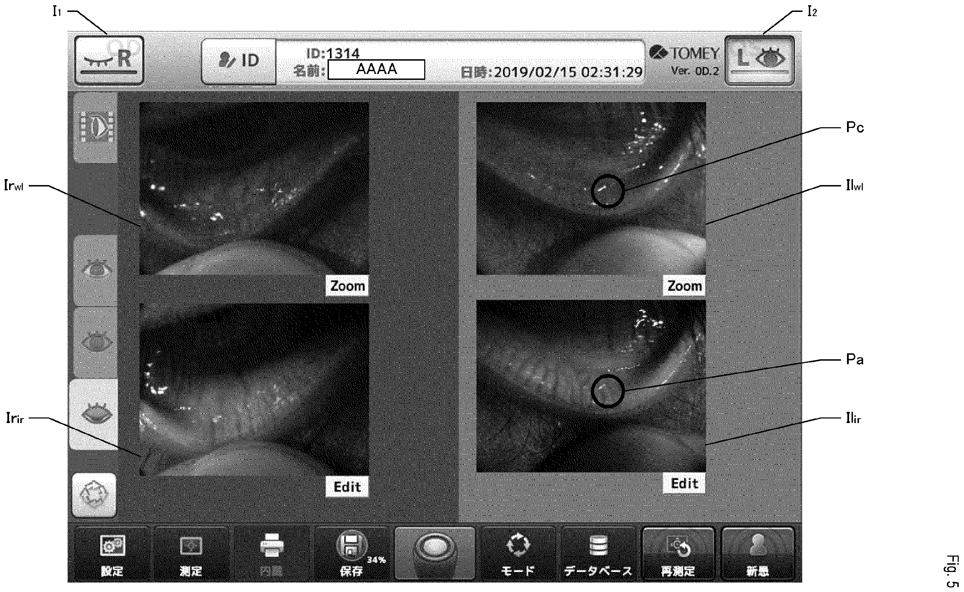

As a result, the infrared image obtained by photographing the eye to be examined under infrared illumination and the white light image obtained by photographing the eye to be examined under white light illumination are simultaneously displayed on the display unit 650. And both the images are obtained by photographing the eye to be examined in the same state. The screen configuration for displaying these images may be in various modes. FIG. 5 is a diagram illustrating a display example of the infrared image and the white light image. In FIG. 5, a configuration is adopted in which an infrared image and a white light image taken for each of the right eye and the left eye of a subject are displayed simultaneously. In FIG. 5, an icon I₁ indicated as R represents the right eye, and an icon I₂ indicated as L represents the left eye. Images of the right eye are shown on the left side of the screen, and images of the left eye are shown on the right side of the screen.

Further, in the example shown in FIG. 5, white light images are displayed at the upper part and infrared images are displayed at the lower part. Accordingly, a region Irwl provided at the upper left is a display region for a white light image of the right eye, and a region Irir provided at the lower left is a display region for an infrared image of the right eye. A region Ilwl provided at the upper right is a display region for a white light image of the left eye, and a region Ilir provided at the lower right is a display region for an infrared image of the left eye. Although not expressed in FIG. 5, actually, the white light image of the right eye is displayed as a color image in the region Irwl, and the infrared image of the right eye is displayed as a monochrome image in the region Irir. Further, the white light image of the left eye is displayed as a color image in the region Ilwl, and the infrared image of the left eye is displayed as a monochrome image in the region Ilir.

According to the above configuration, the examiner can easily visually recognize meibomian glands based on the infrared images displayed in the regions Irir and Ilir, and can identify a region of interest which is suspected of having abnormality. In this embodiment, since the white light images are displayed in the regions Irwl and Ilwl adjacent to the regions Irir and Ilir, respectively, where the infrared images are displayed, the region of interest identified based on the infrared image can be easily contrasted in the white light image.

### (3) Region-of-interest observation processing:

This embodiment has a configuration for enabling easier execution of such contrast. Specifically, this embodiment has a configuration of using the infrared image as an image of interest and the white light image as a contrast image, acquiring a region of interest in the infrared image as the image of interest, and displaying a corresponding region corresponding to the region of interest on the white light image as the contrast image.

The processing for the contrast is realized by the control unit 600 executing a control program (not shown). Specifically, when the examiner gives an instruction to start the region-of-interest observation processing using the touch panel 660 or the like, the control unit 600 executes the control program and functions as a region-of-interest acquisition unit 600c. Then, the control unit 600 executes the region-of-interest observation processing shown in FIG. 4B by the function of the region-of-interest acquisition unit 600c to perform the contrast.

Specifically, when the region-of-interest observation processing is started, the control unit 600 accepts designation of a region of interest by the function of the region-of-interest acquisition unit 600c (step S200). That is, the control unit 600 accepts an examiner's operation of the joystick 640 and/or the touch panel 660, and accepts designation of the region of interest in the infrared image. In this embodiment, the region of interest is an abnormal region of a meibomian gland. Therefore, the examiner operates the joystick 640 and/or the touch panel 660 to identify a region suspected of having a meibomian gland abnormality, and designates the region on the infrared image.

Next, the control unit 600 displays the region of interest (step S205). That is, the control unit 600 controls the display unit 650 to display an icon representing the region of interest at the region of interest accepted in step S200. FIG. 5 shows a state in which a region of interest Pa is represented by a circular icon on the infrared image of the left eye displayed in the region Ilir.

Next, the control unit 600 identifies a corresponding region corresponding to the accepted position by the function of the display control unit 600b (step S210). That is, both the infrared image and the white light image are taken by the two-dimensional image sensor 306, and the respective images are taken almost simultaneously. Therefore, the image present at a specific position on the infrared image is also present at the same position on the white light image. Therefore, the control unit 600 assumes that the coordinates of the region of interest designated in step S200 are images of the corresponding region.

Next, the control unit 600 displays the corresponding region (step S215). That is, control unit 600 controls the display unit 650 to display an icon representing the corresponding region at the corresponding region identified in step S210. FIG. 5 shows a state in which a corresponding region Pc is represented by a circular icon on the white light image of the left eye displayed in the region Ilwl. According to the above configuration, the corresponding region corresponding to the abnormal region of the meibomian gland identified by the infrared image is clearly indicated on the white light image. Therefore, the examiner can easily identify the corresponding region corresponding to the abnormal region on the white light image. Since the colors of the eye to be examined in the white light image and under white light are almost the same, the examiner can easily identify the region suspected of having a meibomian gland abnormality on the actual eye to be examined, based on the corresponding region indicated in the white light image.

### (4) Other Embodiments:

The above embodiment is an example for carrying out the present invention, and various other embodiments can be adopted as long as an infrared image and a white light image showing the eye to be examined in the same state are taken and displayed. For example, the ophthalmic device is not limited to the configuration including the head part and the main body part as described above, and may be an ophthalmic device additionally including other various elements.

The infrared photographing optical system only needs to be able to illuminate the eye to be examined with infrared rays and take an infrared image. Specifically, the infrared photographing optical system includes a light source that outputs infrared rays, a sensor that images infrared rays reflected by the eye to be examined, and an optical component between the light source and the sensor. Of course, the infrared photographing optical system may employ various configurations other than the embodiment described above. For example, in the embodiment described above, the infrared photographing optical system and the white light photographing optical system share components other than the light sources, but may share any other component or all components. In the latter, a light source capable of outputting infrared rays and white light is used, and light is selected by a filter or the like for photographing. Further, the infrared photographing optical system and the white light photographing optical system may be different optical systems.

The white light photographing optical system only needs to be able to illuminate the eye to be examined with white light and take a white light image. Specifically, the white light photographing optical system includes a light source that outputs white light, a sensor that images white light reflected by the eye to be examined, and an optical component between the light source and the sensor. Of course, the white light photographing optical system may employ various configurations other than the embodiment described above.

The photographing control unit only needs to be able to control the infrared photographing optical system and the white light photographing optical system to take an infrared image and a white light image showing the eye to be examined in the same state. In other words, the photographing control unit can execute control necessary for photographing on the target to be controlled. The control necessary for photographing includes, for example, at least one of: alignment of the ophthalmic device; a focusing operation by driving an optical component; acquisition of a taken image by driving a sensor; image processing of a taken image; and storage of a taken image.

The states of the eye to be examined have only to be states observed by the examiner, i.e., states which are similar enough to identify the same region in the eye to be examined when the infrared image and the white light image are contrasted with each other. Therefore, the states in which the eye to be examined looks almost the same are also the same state. For example, when the eye to be examined is photographed simultaneously or almost simultaneously and an infrared image and a white light image are obtained, it can be said that images in the same state are taken.

Photographing almost simultaneously refers to a state in which an infrared image and a white light image are taken in a short period of time such that the corresponding regions can be identified by contrasting the eyes to be examined on these images. There can be various states other than the state in which the infrared image and the white light image are taken at timings different by one frame, as in the embodiment described above. For example, even a state in which an infrared image and a white light image are taken at timings different by several frames is a state in which these images are taken almost simultaneously. Further, even when an infrared image and a white light image are taken within a predetermined interval in the state in which the subject is instructed not to move, the corresponding portions in both the images can be identified by contrasting these images. Therefore, it can be said that these images are taken in the same state.

The display control unit only needs to be able to cause the display unit to display each of the infrared image and the white light image. That is, the display control unit only needs to be configured to cause the display unit to display the infrared image and the white light image to contrast these images with each other. Various modes in which the infrared image and the white light image are displayed on the display unit may be adopted. Therefore, various configurations can be adopted other than the configuration in which the infrared images and white light images of the right and left eyes are simultaneously displayed on one screen, as in the embodiment described above. For example, the display unit is not limited to one screen, and may include a plurality of screens. The display unit may be configured to be connected to the ophthalmic device with a cable or the like, instead of being integrated with the ophthalmic device. In addition, the infrared image and the white light image may be output from the ophthalmic device to any other device (for example, a general-purpose computer), and displayed on a display unit connected to the other device.

The region-of-interest acquisition unit only needs to be able to define one of the infrared image and the white light image as an image of interest and the other thereof as a contrast image, and acquire the region of interest existing on the image of interest. That is, the region of interest may be acquired from the white light image, or may be acquired from both the infrared image and the white light image. In the latter case, both the infrared image and the white light image are images of interest and are also contrast images. Further, the region of interest is not limited to the abnormal region of the meibomian gland. For example, it is also possible to take and display an infrared image in which an iris is easily recognized by infrared rays, and to take and display a white light image in which blood vessels are easily recognized by white light.

As the configuration for the region-of-interest acquisition unit to acquire the region of interest, various configurations can be adopted other than the configuration in which the region-of-interest is acquired by designation of the examiner, as in the embodiment described above. For example, it is possible to adopt a configuration of performing image processing based on at least one of the infrared image and the white light image and acquiring features of the target of interest to acquire the region of interest. The image processing for acquiring the features may be various types of processing, such as pattern matching or the like, or artificial intelligence processing using machine learning or the like.

Furthermore, the infrared image and the white light image may be displayed after various types of image processing are performed. The image processing includes, for example, enhancement processing that emphasizes the region of interest. Various enhancement techniques can be employed, and examples thereof include contrast enhancement, brightness enhancement, intermediate gradation color enhancement, edge enhancement, and various types of filter processing. The image to be processed may be either the infrared image or the white light image, and may be either the right eye image or the left eye image.

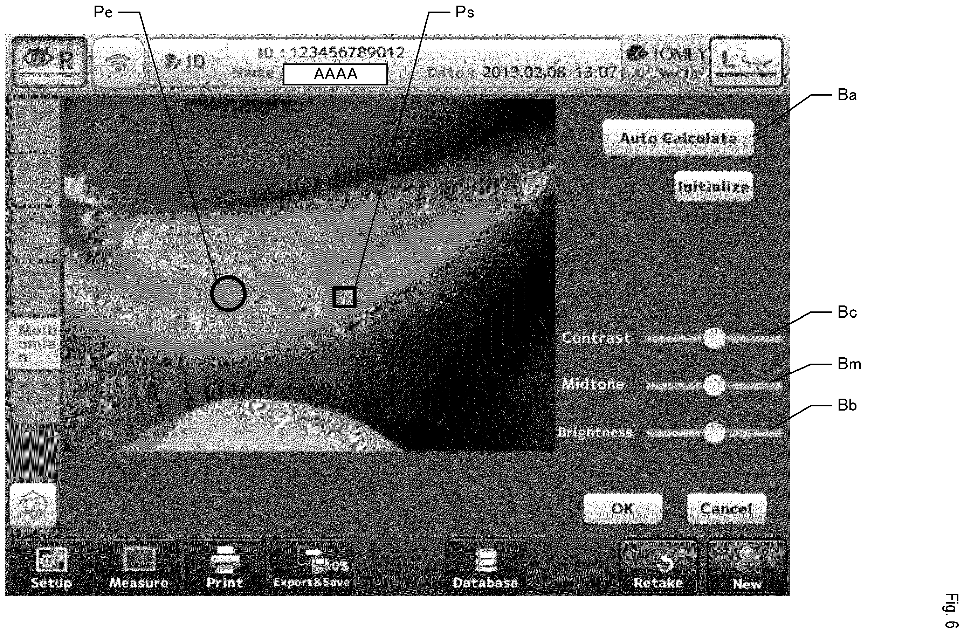

FIG. 6 shows a screen for performing various types of image processing. FIG. 6 shows a state in which an infrared image of the right eye is displayed as the image to be processed. For example, in the configuration shown in FIG. 3, when the examiner gives an instruction to start image processing and an instruction that the infrared image for the right eye is to be imaged, using the joystick 640 and/or the touch panel 660, this image is displayed on the display unit 650 by the control of the control unit 600. The image displayed in the example shown in FIG. 6 is an image obtained by enlarging the image shown in FIG. 5.

The screen shown in FIG. 6 includes a contrast adjustment part Bc, an intermediate gradation color adjustment part Bm, and a brightness adjustment part Bb. The examiner can instruct the amount of the contrast, intermediate gradation color, and brightness to be adjusted by moving the buttons of the adjustment parts laterally using the joystick 640 and/or the touch panel 660. Based on the instruction, the control unit 600 executes contrast, intermediate gradation color and brightness enhancement processing (or non-enhancement processing), and causes the display unit 650 to display the image after execution.

In the example shown in FIG. 6, there is a function of automatically adjusting the image quality in addition to the adjustment by the examiner. In this example, a reference region for adjusting the image quality is acquired, and an image is enhanced based on image information of the region. That is, the examiner operates the joystick 640 and/or the touch panel 660 while the screen shown in FIG. 6 is displayed, and instructs a reference region on the image. The control unit 600 identifies a reference region based on the instruction. In FIG. 6, the reference region is indicated as a region Ps.

When the reference region is instructed, the examiner performs an operation on an automatic enhancement button Ba using the joystick 640 and/or the touch panel 660. As a result, the control unit 600 performs contrast enhancement so that the luminance range of the pixels included in the reference region is distributed over a wider luminance range (for example, the entire range of luminance that can be expressed). The range to which the contrast enhancement is applied may be the entire range of the image or a partial range. In the latter case, a configuration in which the range is instructed by the examiner is exemplified. For example, a configuration may be employed in which the examiner operates the joystick 640 and/or the touch panel 660 to select an enhancement range Pe on the screen shown in FIG. 6, and the control unit 600 applies contrast enhancement to the selected range Pe. Of course, the type of image processing is not limited to contrast enhancement, and may be various other types of processing such as brightness enhancement.

Furthermore, a technique of taking and displaying an infrared image and a white light image showing the eye to be examined in the same state can also be applied as a method invention. The ophthalmic device and method as described above can be realized as a single apparatus or as a part of an apparatus having a plurality of functions, and includes various modes.

### Reference Signs List

1 ophthalmic device
100 alignment optical system
101 light source
102 hot mirror
103 objective lens
104 hot mirror
105 lens
106 lens
107 profile sensor
108 profile sensor
200 displacement/deformation detection light receiving optical system
201 light source
202 half mirror
203 condenser lens
204 light receiving element
205 nozzle
206 flat glass
300 observation optical system
301a, 301b, 302a, 302b light source
303 objective lens
304 dichroic mirror
305 imaging lens
306 two-dimensional image sensor
400 fixation optical system
401 light source
402 hot mirror
403 relay lens
404 reflection mirror
500 eye refractive power optical system
501 light source
502 condenser lens
503 mirror
504 mirror
505 parallel flat glass
506 hot mirror
507 lens
508 ring lens
509 CCD
510 flat glass
511 flat glass
512 fixation target
513 collimator lens
514 light source
600 control unit
600a photographing control unit
600b display control unit
600c region-of-interest acquisition unit
601 main body part
602 head part
630 XYZ drive control unit
640 joystick
650 display unit
660 touch panel
670 memory
680 fixation target control unit
FIGS. 1, 2
   EYEPIECE PART
FIG. 3
   602 HEAD PART
   100 ALIGNMENT OPTICAL SYSTEM
   300 OBSERVATION OPTICAL SYSTEM
   400 FIXATION OPTICAL SYSTEM
   200 DISPLACEMENT/DEFORMATION DETECTION LIGHT RECEIVING OPTICAL SYSTEM
   500 EYE REFRACTIVE POWER OPTICAL SYSTEM
   600 CONTROL UNIT
   600a PHOTOGRAPHING CONTROL UNIT
   600b DISPLAY CONTROL UNIT
   600c REGION-OF-INTEREST ACQUISITION UNIT
   601 MAIN BODY PART
   630 XYZ DRIVE CONTROL UNIT
   640 JOYSTICK
   650 DISPLAY UNIT
   660 TOUCH PANEL
   670 MEMORY
   680 FIXATION TARGET CONTROL UNIT
FIG. 4A
   PHOTOGRAPHING/DISPLAY PROCESSING
   S100 DETECT POSITION OF EYE TO BE EXAMINED
   S105 MOVE TO REFERENCE POSITION
   S110 REFERENCE POSITION?
   S115 TAKE INFRARED IMAGE AND WHITE LIGHT IMAGE WHILE MOVING LENS POSITION CONTINUOUSLY
   S120 ACQUIRE INFRARED IMAGE AND WHITE LIGHT IMAGE WITH HIGHEST CONTRAST
   S125 DISPLAY INFRARED IMAGE AND WHITE LIGHT IMAGE
FIG. 4B
   REGION-OF-INTEREST OBSERVATION PROCESSING
   S200 ACCEPT DESIGNATION OF REGION OF INTEREST
   S205 DISPLAY REGION OF INTEREST
   S210 IDENTIFY CORRESPONDING REGION CORRESPONDING TO ACCEPTED POSITION
   S215 DISPLAY CORRESPONDING REGION

## Claims

1. An ophthalmic device (1) comprising:
an infrared photographing optical system that illuminates an eye to be examined with infrared rays to take an infrared image;
a white light photographing optical system that illuminates the eye to be examined with white light to take a white light image;
a photographing control unit (600a) that controls the infrared photographing optical system and the white light photographing optical system to take the infrared image and the white light image indicating the eye to be examined in a same state; and
a display control unit (600b) that displays each of the infrared image and the white light image on a display unit (650) .

2. The ophthalmic device (1) according to claim 1, wherein the infrared photographing optical system and the white light photographing optical system share at least a part of components.

3. The ophthalmic device (1) according to claim 1 or 2, wherein the display control unit (600b):
causes the display unit (650) to display the infrared image as a monochrome image; and
causes the display unit (650) to display the white light image as a color image.

4. The ophthalmic device (1) according to any one of claims 1 to 3, further comprising a region-of-interest acquisition unit (600c) that defines one of the infrared image and the white light image as an image of interest and the other thereof as a contrast image, and acquires a region of interest existing on the image of interest,
wherein the display control unit (600b) displays a corresponding region corresponding to the region of interest on the contrast image.

5. The ophthalmic device (1) according to claim 4, wherein the image of interest is the infrared image, the contrast image is the white light image, and the region of interest is an abnormal region of a meibomian gland.

6. The ophthalmic device (1) according to claim 4 or 5, wherein the region-of-interest acquisition unit (600c) acquires a region designated by an examiner on the image of interest as the region of interest.
